# EUROPEAN PATENT APPLICATION

(11) **EP 2 251 431 A1**
(43) Date of publication of application: **17.11.2010**
(21) Application number: 09707649.1
(22) Date of filing: 05.02.2009
(51) Int. Cl.: C12P 41/00

(54) **PROCESS FOR PRODUCTION OF OPTICALLY ACTIVE INDOLINE-2-CARBOXYLIC ACID OR DERIVATIVE THEREOF**

(30) Priority: 06.02.2008 JP 2008026115
(71) Applicant: Mitsubishi Gas Chemical Company, Inc., Chiyoda-ku Tokyo 100-8324 (JP)
(72) Inventor: IIDA, Akifumi, Niigata-shi Niigata 950-3112 (JP); KYUUKO, Youichi, Niigata-shi Niigata 950-3112 (JP)
(74) Representative: Harrison, Robert John
(86) International application number: PCT/JP2009/051957
(87) International publication number: WO 2009/099140

(57) **Abstract**

Disclosed is a process for producing an optically active indoline-2-cabroxylic acid or a derivative thereof, which is useful as a raw material for synthesis of pharmaceuticals or the like, from an indoline-2-cabroxylic acid in an industrially advantageous manner.

An indoline-2-cabroxylic acid is converted into an N-substituted-indoline-2-carboxylic acid, and then is esterified using a biocatalyst having a stereoselectivity in an organic solvent containing an alcohol. Next, an alkali is used to obtain an optically active N-substituted-indoline-2-carboxylic acid ester and an optically active N-substituted-indoline-2-cabroxylic acid or a salt thereof, and then the two optically active substances are separated utilizing distribution properties thereof in an organic solvent/water system. Further, the two substances are hydrolyzed and deprotected, whereby the optically active indoline-2-cabroyxlic acid or a derivative thereof, which is useful as a raw material for synthesis of pharmaceuticals or the like, can be produced and provided economically.

## Description

### TECHNICAL FIELD

The present invention relates to a process for efficiently producing an optically active indoline-2-carboxylic acid or a derivative thereof, which is useful as a raw material for synthesis of pharmaceuticals or the like. That is, more specifically, it relates to a process for producing an optically active indoline-2-carboxylic acid, an optically active indoline-2-carboxylic acid ester, an optically active N-substituted-indoline-2-carboxylic acid or an optically active N-substituted-indoline-2-carboxylic acid ester excellent in both chemical purity and optical purity from an indoline-2-carboxylic acid shown in the formula (1) in high yield. These optically active indoline-2-carboxylic acids and derivatives thereof are very important as raw materials for synthesis of pharmaceuticals and others that require optical activity. 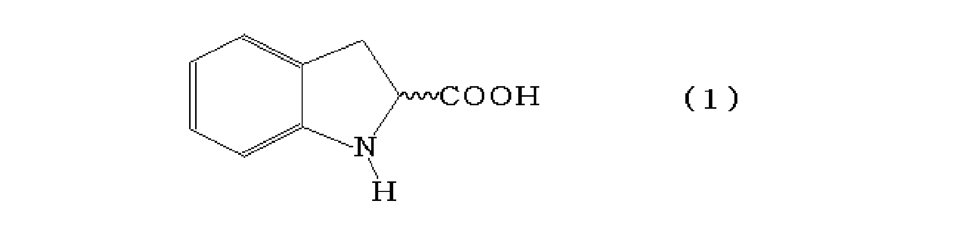 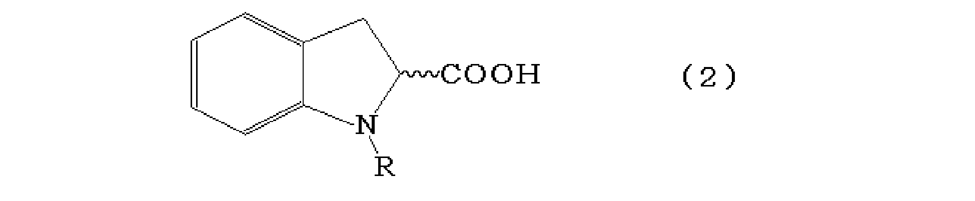 wherein the substituent R represents a formyl group, acetyl group, methoxycarbonyl group, tert-butoxycarbonyl group or benzyloxycarbonyl group.

### BACKGROUND ART

Methods for synthesizing an optically active indoline-2-carboxylic acid include (1) a method for enantioselectively hydrolyzing an optically active indoline-2-carboxylic acid ester using a biocatalyst (for example, refer to Non-Patent Document 1), (2) an optical resolution method in accordance with a diastereomer method using an optically active amine (for example, refer to Patent Documents 1 and 2), and (3) a synthesis method using an asymmetric ligand (for example, refer to non-Patent Document 2). (1) is a method in which the reaction is performed in an aqueous system and thus is slow in reaction rate and low in reaction yield. (2) is a method in which the optically active amine used as an optical resolving reagent is costly, and the step of recovering and purifying the amine is complicated and encumbers industrialization. (3) is a method which is still under investigation, and has many problems such that lots of ligands must used and production process of ligands is complicated.

Patent Document 1: Japanese Patent Laid-open No. 2004-182670
Patent Document 2: Japanese Patent Laid-open No. H11-292844
Non-Patent Document 1: Takashi Sugai, et al., Bull. Chem. Jpn., 77, 1021-1025 (2004)
Non-Patent Document 2: Yoshihiko Ito, et al., J. Am. Chem. Soc., 122, 7614-7615 (2005)

### DISCLOSURE OF THE INVENTION

### PROBLEM TO BE SOLVED BY THE INVENTION

The present invention aims at solving the problems of conventional techniques as mentioned above, and providing a simple and industrially advantageous process for producing an optically active indoline-2-carboxylic acid or a derivative thereof.

### MEANS FOR SOLVING THE PROBLEM

The present inventors have studied intensively in order to solve such problems, and reached the present invention. That is, they have found that when the N-substituted-indoline-2-carboxylic acid shown in the formula (2) which results from introduction of a substituent into the nitrogen of the indoline-2-carboxylic acid shown in the formula (1) is used as a substrate and allowed to react with a biocatalyst having a stereoselectivity in an organic solvent containing an alcohol, the carboxyl group of the indoline-2-carboxylic acid can be esterified in good yield with extremely high stereoselectivity compared with the case where the indoline-2-carboxylic acid shown in the formula (1) is directly used as a substrate; the distribution of the resulting optically active N-substituted-indoline-2-carboxylic acid ester and the other enantiomeric optically active N-substituted-indoline-2-carboxylic acid salt between an organic solvent layer and an aqueous layer becomes distinct compared with the distribution of the optically active indoline-2-carboxylic acid ester and the optically active indoline-2-carboxylic acid salt, and thereby makes it easier to separate the optically active substances from each other; and hydrolysis and deprotection of the resulting optically active N-substituted-indoline-2-carboxylic acid ester and the optically active N-substituted-indoline-2-carboxylic acid (salt) can provide an optically active indoline-2-carboxylic acid including an optically active indoline-2-carboxylic acid (salt) and an optically active N-substituted-indoline-2-carboxylic acid (salt), or an optically active indoline-2-carboxylic acid derivative including an optically active indoline-2-carboxylic acid ester and an optically active N-substituted-indoline-2-carboxylic acid ester in high yield without lowering optical purity. Thus, they have completed the present invention.

That is, the present invention relates to a process for producing an optically active indoline-2-cabroxylic acid or a derivative thereof with good quality and yield from the indoline-2-carboxylic acid shown in the formula (1), as indicated in the following items 1-10.
1. A process for producing an optically active indoline-2-carboxylic acid or a derivative thereof from an indoline-2-carboxylic acid shown in the formula (1), which comprises the following steps (A) to (B), the following steps (A) to (C), the following steps (A) to (D) or the following steps (A), (B) and (E):
   Step (A): a step in which an indoline-2-carboxylic acid shown in the formula (1) is converted into an N-substituted-indoline-2-cabroxylic acid shown in the formula (2), and then is esterified using a biocatalyst having a stereoselectivity in an organic solvent containing an alcohol, to obtain a composition comprising an optically active N-substituted-indoline-2-carboxylic acid ester and the other enantiomeric optically active N-substituted-indoline-2-caroboxylic acid which is not esterified;
   Step (B): a step in which the composition obtained in the step (A) is mixed with an organic solvent and a basic substance in a presence of water, and allowed to separate into two layers to collect an organic solvent layer containing the optically active N-substituted-indoline-2-carboxylic acid ester and an aqueous layer containing the optically active N-substituted-indoline-2-cabroxylic acid or a salt thereof;
   Step (C): a step in which the optically active N-substituted-indoline-2-carboxylic acid ester obtained from the organic solvent layer collected in the step (B) is hydrolyzed to obtain an optically active N-substituted-indoline-2-carboxylic acid;
   Step (D): a step in which the N-substituent of the optically active N-substituted-indoline-2-carboxylic acid obtained in the step (C) is deprotected to obtain an optically active indoline-2-cabroxylic acid; and
   Step (E): a step in which the N-substituent of the optically active N-substituted-indoline-2-cabroxylic acid or a salt thereof obtained from the aqueous layer collected in the step (B) is deprotected to obtain an optically active indoline-2-cabroxylic acid; 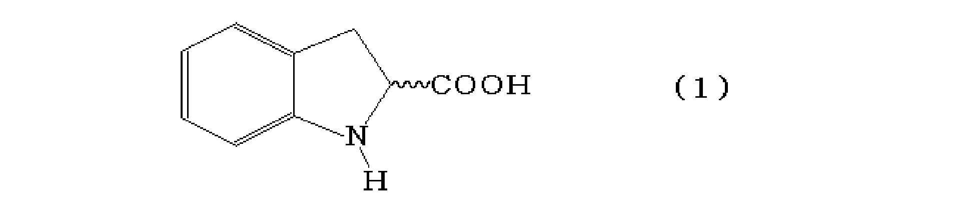 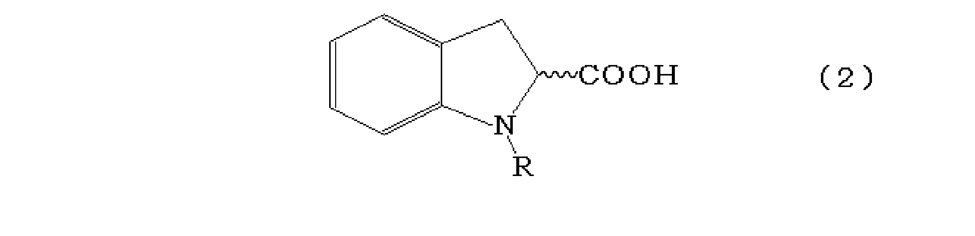 wherein the substituent R represents a formyl group, acetyl group, methoxycarbonyl group, tert-butoxycarbonyl group or benzyloxycarbonyl group.
2. The process for producing an optically active indoline-2-carboxylic acid or a derivative thereof, according to item 1, wherein the biocatalyst having a stereoselectivity is an esterase.
3. The process for producing an optically active indoline-2-carboxylic acid or a derivative thereof, according to item 2, wherein the esterase is a lipase.
4. The process for producing an optically active indoline-2-carboxylic acid or a derivative thereof, according to item 3, wherein the lipase is derived from an yeast which belongs to the genus Candida.
5. The process for producing an optically active indoline-2-carboxylic acid or a derivative thereof, according to item 1, wherein the alcohol is a primary or secondary alcohol.
6. The process for producing an optically active indoline-2-carboxylic acid or a derivative thereof, according to item 5, wherein the primary or secondary alcohol is one or more selected form the group consisting of methanol, ethanol, 1-propanol, 2-propanol, 1-butanol, 2-butanol, 2-methyl-1-propanol, 1-pentanol, 2-pentanol and 3-pentanol.
7. The process for producing an optically active indoline-2-carboxylic acid or a derivative thereof, according to item 5, wherein the alcohol is a methanol.
8. The process for producing an optically active indoline-2-carboxylic acid or a derivative thereof, according to item 1, wherein the organic solvent is one or more selected from the group consisting of diisopropyl ether, n-butyl ether and ethyl acetate.
9. The process for producing an optically active indoline-2-carboxylic acid or a derivative thereof, according to 1, which further comprises a step of subjecting the optically active indoline-2-cabroxylic acid or a salt thereof obtained in the step (D) or (E) to an esterification or N-substitution reaction.
10. The process for producing an optically active indoline-2-carboxylic acid or a derivative thereof, according to any one of items 1 to 9, wherein the optically active indoline-2-carboxylic acid derivative is an optically active indoline-2-carboxylic acid ester or an optically active N-substituted-indoline-2-cabroxylic acid ester.

### EFFECT OF THE INVENTION

According to the present invention, an optically active indoline-2-carboxylic acid and a derivative thereof can be produced efficiently, which are useful as raw materials for synthesis of pharmaceuticals or the like, and are excellent in both chemical purity and optical purity.

### BEST MODE FOR CARRYING OUT THE INVENTION

Hereinafter, the present invention will be described in detail.

In the present invention, an N-substituted-indoline-2-cabroxylic acid shown in the formula (2) resulting from introduction of a substituent R into the nitrogen site of the indoline-2-cabroxylic acid shown in the formula (1) is used. The use as a substrate of the substance resulting from the conversion into an N-substituted-indoline-2-cabroxylic acid enables the stereoselective esterification using a biocatalyst to proceed with extremely high selectivity and high yield.

The substituent R introduced into the nitrogen site of the indoline-2-cabroxylic acid is preferably a formyl group, acetyl group, methoxycarbonyl group, tert-butoxycarbonyl group or benzyloxycarbonyl group, and particularly preferably an acetyl group.

As the method for introducing the substituent R into the nitrogen site, a method using acetic anhydride, benzyloxycarbonyl chloride, di-tert-butylcarbonate or the like under a basic condition is generally well-known. For example, a formyl-substituted compound can be obtained by reaction with formic acid, an acetyl-substituted compound can be obtained by reaction with acetic anhydride, a methoxycarbonyl-substituted compound can be obtained by reaction with methyl chloroformate, a tert-butoxycarbonyl-substituted compound can be obtained by reaction with di-tert-butyldicarbonate, and a benzyloxycarbonyl-substituted compound can be obtained by reaction with benzyl chloroformate.

Meanwhile, since a compound is often used for synthesis of pharmaceuticals with its reactive nitrogen site or carboxyl group being protected, the optically active N-substituted-indoline-2-carboxylic acid ester obtained in the above step (B) of the present invention and the optically active N-substituted-indoline-2-cabroxylic acid or a salt thereof obtained in the above step (B) or (C) of the present invention can also be utilized as raw materials for synthesis of pharmaceuticals and the like.

The biocatalyst used in the present invention may be one capable of stereoselectively esterifying either enantiomer of the N-substituted-indoline-2-caorboxylic acid in an organic solvent containing an alcohol, and is not particularly limited in origin. Examples of the biocatalyst having such a capability include an esterase such as a lipase derived from a microorganism, that is, for example, one derived from a microorganism which belongs to the genus Candida, Aspergillus, Alcaligenes or Pseudomonas, and among them, a preferable example thereof includes a lipase derived from an yeast which belongs to the genus Candida, particularly a lipase which is produced by Candida antarctica. Meanwhile, forms of the lipase are not particularly limited, and microorganism cells containing the lipase, the lipase itself, the lipase immobilized to a carrier, or the like can be used.

The alcohol contained in the organic solvent used in the present invention includes a primary or secondary alcohol, and concretely methanol, ethanol, 1-propanol, 2-propanol, 1-butanol, 2-butanol, 2-methyl-1-propanol, 1-pentanol, 2-pentanol or 3-pentanol is used. Above all, preferable one is methanol or ethanol, and more preferably methanol.

The organic solvent used in the present invention may be preferably one which does not prevent the present reaction using the biocatalyst, well dissolves the N-substituted-indoline-2-carboxylic acid as a substrate and the optically active N-substituted-indoline-2-cabroxylic acid ester as a product, separates from the aqueous layer, and has an appropriate boiling point. Such a solvent includes aliphatic hydrocarbons such as n-hexane, n-heptane and isooctane, aromatic hydrocarbons such as benzene and toluene, ethers such as dimethyl ether, diethyl ether, diisopropyl ether, tert-butyl ether and n-butyl ether, and esters such as methyl acetate and ethyl acetate. These organic solvents can be used alone or in combination of two or more. Meanwhile, moisture content in the organic solvent is desired to be smaller in order to conduct the esterification at a better conversion, and is preferably not more than 1.0 weight% and more preferably not more than 0.5 weight%.

Concentration of the N-substituted-indoline-2-cabroxylic acid as a substrate in the reaction solution is preferably 0.5-20 weight% and more preferably 1-10 weight%. Meanwhile, molar ratio of the alcohol relative to the N-substituted-indoline-2-cabroxylic acid is preferably 1 to 10 times and more preferably 1 to 5 times on molar basis. In addition, the type of reaction such as batch type and continuous type is not limited in the present esterification, and any type of reaction can be conducted.

By the way, the biocatalyst has an activity which differs depending upon production lots, and often differs depending upon forms thereof. The amount of lipase to be used can be arbitrarily determined so as to suit an aimed reaction yield or reaction time according to the activity of the lipase to be used.

Since a biocatalyst requires a temperature range suitable for a reaction, a suitable reaction temperature must be selected according to the biocatalyst and the composition of the reaction solution to be used. The preferable reaction temperature for the present invention is 30-90°C. When the reaction temperature is lower than 30°C, a sufficient reaction rate cannot be obtained, and when it is higher than 90°C, the activity of the biocatalyst is lowered due thermal denaturation which further leads to lowering of the reaction rate disadvantageously. Generally, heat resistance can be given to the biocatalyst by immobilizing it to a carrier, and thus this is useful for improving the reaction rate.

The resulting optically active N-substituted-indoline-2-carboxylic acid ester and the optically active N-substituted-indoline-2-carboxylic acid which is not esterified can be separated by extraction-separation using an organic solvent. That is, when the optically active N-substituted-indoline-2-carboxylic acid is converted into a salt with an alkali metal such as sodium, it is lowered in solubility in the organic solvent and is increased in solubility in water. Utilizing this property, only the optically active N-substituted-indoline-2-carboxylic acid which is not stereoselectively esterified but is in a form of sodium salt can be transferred to the aqueous layer and separated from the stereoselectively esterified optically active N-substituted-indoline-2-carboxylic acid ester which exists in the organic solvent, when an aqueous sodium carbonate solution or the like is added to the reaction solution after the completion of stereoselective esterification.

The optically active N-substituted-indoline-2-carboxylic acid ester in the organic solvent layer can be isolated by distilling the organic solvent. Also, a sodium salt of the optically active N-substituted-indoline-2-carboxylic acid which exists in the aqueous layer may be treated with an aqueous acid solution such as hydrochloric acid to yield the optically active N-substituted-indoline-2-carboxylic acid which may further be purified, if required.

The resulting optically active N-substituted-indoline-2-carboxylic acid ester can be heated in an alkali aqueous solution such as sodium hydroxide to be hydrolyzed and yield the optically active N-substituted-indoline-2-carboxylic acid without racemization.

The optically active N-substituted-indoline-2-carboxylic acid can be subjected to removal of the substituent in accordance with a known method to yield the optically active indoline-2-carboxylic acid without racemization. The respective substituents can be easily removed, for example, the formyl substituent can be removed by reaction with sodium hydroxide, the acetyl substituent can be removed by reaction with hydrochloric acid, the methoxycarbonyl substituent can be removed by reaction with trifluoroacetic acid, the tert-butoxycarbonyl substituent can be removed by reaction with trifluoroacetic acid, and the benzyloxycarbonyl substituent can be removed by catalytic hydrogenation. Meanwhile, the optically active indoline-2-carboxylic acid ester can be obtained by removing the substituent from the optically active N-substituted-indoline-2-carboxylic acid ester.

As described above, the targeted optically active indoline-2-carboxylic acid and a derivative thereof excellent in chemical purity and optical purity can be produced in high yield by conducting a stereoselective biocatalytic reaction using, in place of the indoline-2-carboxylic acid, an N-substituted-indoline-2-carboxylic acid resulting from introduction of a substituent into the nitrogen atom of the indoline-2-carboxylic acid, as a substrate.

Meanwhile, the optically active indoline-2-carboxylic acid obtained in the above step (D) or (E) of the present invention may be further subjected to an esterification or N-substitution reaction step so as to obtain an optically active indoline-2-carboxylic acid ester, an optically active N-substituted-indoline-2-carboxylic acid ester, an optically active N-substituted-indoline-2-carboxylic acid or a derivative thereof.

### EXAMPLE

Hereinafter, the present invention will be described in more detail by way of Examples and Comparative Examples. However, the present invention is in no way restricted to these Examples.

Meanwhile, optical purity of optically active N-substituted-indoline-2-cabroxylic acid esters, optically active N-substituted-indoline-2-cabroxylic acid and the like was measured by high performance liquid chromatography (HPLC). As a biocatalyst having a stereoselectivity, CHIRAZYME L-2, c-f, C2 (manufactured by Roche Diagnostics K.K.) in which a lipase derived from Candida antarctica was immobilized to a carrier was used.

### Example 1

### Production of (S)-indoline-2-carboxylic acid and (R)-indoline-2-carboxylic acid from indoline-2-carboxylic acid

### 1. Production of (S)-N-acetylindoline-2-carboxylic acid methyl ester and (R)-N-acetylindoline-2-carboxylic acid

3 g of a racemic N-acetylindoline-2-carboxylic acid obtained by introducing an acetyl group to the nitrogen atom of a racemic indoline-2-cabroxylic acid, 54 g of n-butyl ether, 1.5 g of methanol and 1.5 g of an immobilized lipase CHIRAZYME L-21, c-f, C2 as a biocatalyst were added to a 200 ml autoclave, and reaction was effected under a stirring condition of 120 rpm at 80°C for 24 hours. According to analysis of the reaction solution with HPLC, the conversion of N-acetylindoline-2-carboxylic acid in the raw materials to (S)-N-acetylindoline-2-carobxylic acid methyl ester was 45.7%, and the optical purity thereof was 98.8%ee. On the other hand, the optical purity of the unreacted (R)-N-acetylindoline-2-carboxylic acid was 83.0%ee.

### 2. Separation of (S)-N-acetylindoline-2-carboxylic acid methyl ester from (R)-N-acetylindoline-2-carboxylic acid

10 ml of methanol was added to the above reaction solution to dissolve the unreacted (R)-N-acetylindoline-2-carboxylic acid which had been precipitated, and then the above immobilized biocatalyst was filtered. Methanol and n-butyl ether in the filtrate were distilled under reduced pressure at a bath temperature of 90C° by rotary evaporator, and then 50 ml of ethyl acetate and 50 ml of a 3% aqueous sodium hydrogen carbonate solution were added to the resulting crystals, and (S)-N-acetylindoline-2-carboxylic acid methyl ester was extracted into the organic layer and (R)-N-acetylindoline-2-carboxylic acid sodium salt into the aqueous layer. Next, ethyl acetate in the collected organic layer was distilled under reduced pressure at a bath temperature of 60C° by rotary evaporator, to obtain 1.45 g (99.0% yield) of (S)-N-acetylindoline-2-carboxylic acid methyl ester with an optical purity of 98.9%ee. On the other hand, the collected aqueous layer was neutralized with a 3M aqueous hydrochloric acid solution, and the precipitated crystals were filtered, to obtain 1.47 g (90.2% yield) of (R)-N-acetylindoline-2-carboxylic acid with an optical purity of 83.1 %ee.

### 3. Production of (S)-N-acetylindoline-2-carboxylic acid

3.0 g of (S)-N-acetylindoline-2-carboxylic acid methyl ester obtained as in 2 above was dissolved in 12 ml of a 2.5 M aqueous sodium hydroxide solution, and stirred at 80 C° for 4 hours under argon atmosphere. After cooling, the reaction solution was neutralized with 12 ml of a 3M aqueous hydrochloric acid solution, and the precipitated crystals were filtered and dried, to obtain 2.26 g (80.5% yield) of (S)-N-acetylindoline-2-carboxylic acid with an optical purity of 99.0%ee.

### 4. Production of (S)-indoline-2-carboxylic acid

3.0 g of (S)-N-acetylindoline-2-carboxylic acid obtained as in 3 above was dissolved in 10 ml of a 3M aqueous hydrochloric acid solution, and stirred at 100 C° for 4 hours to remove the acetyl group. After cooling, the reaction solution was adjusted to pH 5.0 with a 50% aqueous sodium hydroxide solution, and the precipitated crystals were filtered and dried, to obtain 1.86 g (78.0% yield) of (S)-indoline-2-carboxylic acid with the optical purity of not less than 99.9%ee.

### 5. Production of (R)-indoline-2-carboxylic acid

3.0 g of (R)-N-acetylindoline-2-carboxylic acid obtained as in 2 above was dissolved in 10 ml of a 3M aqueous hydrochloric acid solution, and stirred at 100 C° for 4 hours to remove the acetyl group. After cooling, the reaction solution was adjusted to pH 5.0 with a 50% aqueous sodium hydroxide solution, and the precipitated crystals were filtered and dried, to obtain 1.86 g (78.0% yield) of (R)-indoline-2-carboxylic acid with an optical purity of 86.0%ee.

### Example 2

0.20 g of a racemic N-tert-butoxycarbonylindoline-2-carboxylic acid obtained by introducing a tert-butoxycarbonyl group into the nitrogen atom of a racemic indoline-2-carboxylic acid, 30 g of isopropyl ether, 0.20 g of methanol, and 0.2 g of CHIRAZYME, L-2, c-f, C2 were added to a 100 ml glass vial, and shaken at 150 rpm in an 80 C° water bath for 24 hours to effect reaction. According to analysis of the reaction solution with HPLC, (S)-N-tert-butoxycarbonylindoline-2-carboxylic acid methyl ester with an optical purity of 99%ee was obtained at a conversion of 47.4%.

Next, as a result of conducting deprotection of the N-substituent and the ester group in the same manner as in the methods 2 to 4 of Example 1, (S)-indoline-2-carboxylic acid with an optical purity of not less than 99.9% was obtained in 61.5% yield from (S)-N-tert-butoxycarbonylindoline-2-carboxylic acid methyl ester.

### Comparative Example 1

20 mg of a racemic indoline-2-cabroxylic acid, 1 g of n-butyl ether, 20 mg of methanol and 20 mg of CHIRAZYME L-2, c-f, C2 were added to a 50 ml glass vial, and shaken at 150 rpm in a 40 C° water bath for 2 hours. According to analysis of the reaction solution with HPLC, (S)-idoline-2-cabroxylic acid was converted to methyl ester at a conversion of 38.1%, and (R)-idoline-2-cabroxylic acid at a conversion of 22.9%. The optical purity of the produced (S)-idoline-2-cabroxylic acid methyl ester was as low as 25.0%ee.

### Example 3

0.2 g of a racemic N-acetylindoline-2-carboxylic acid obtained in the same manner as in Example 1, 30 g of isopropyl ether, 0.2 g of ethanol, and 0.2 g of CHIRAZYME L-2, c-f, C2 were added to a 100 ml glass vial, and shaken at 150 rpm in an 80 C° water bath for 24 hours. According to analysis of the reaction solution with HPLC, (S)-N-acetylindoline-2-carboxylic acid ethyl ester with an optical purity of 99%ee was obtained at a conversion of 20.9%.

Next, as a result of conducting deprotection of the N-substituent and the ester group in the same manner as in the methods 2 to 4 of Example 1, (S)-indoline-2-carboxylic acid with an optical purity of not less than 99.9% was obtained in 60.8% yield from (S)-N-acetylindoline-2-carboxylic acid ethyl ester.

### Comparative Example 2

20 mg of a racemic indoline-2-cabroxylic acid, 1.0 g of isopropyl ether, 20 mg of ethanol and 20 mg of CHIRAZYME L-2, c-f, C2 were added to a 50 ml glass vial, and shaken at 150 rpm in a 40 C° water bath for 2 hours. According to analysis of the reaction solution with HPLC, (S)-idoline-2-cabroxylic acid was converted to ethyl ester at a conversion of 17.1%, and (R)-idoline-2-cabroxylic acid at a conversion of 10.2%. The optical purity of the produced (S)-idoline-2-cabroxylic acid ethyl ester was as low as 25.3%ee.

### INDUSTRIAL APPLICABILITY

The present invention is useful for producing, in high purity and with high efficiency, an optically active indoline-2-cabroxylic acid or a derivative thereof which is useful as a raw material for synthesis of pharmaceuticals or the like.

## Claims

1. A process for producing an optically active indoline-2-carboxylic acid or a derivative thereof from an indoline-2-carboxylic acid shown in the formula (1), which comprises the following steps (A) to (B), the following steps (A) to (C), the following steps (A) to (D) or the following steps (A), (B) and (E):
Step (A): a step in which an indoline-2-carboxylic acid shown in the formula (1) is converted into an N-substituted-indoline-2-cabroxylic acid shown in the formula (2), and then is esterified using a biocatalyst having a stereoselectivity in an organic solvent containing an alcohol, to obtain a composition comprising an optically active N-substituted-indoline-2-carboxylic acid ester and the other enantiomeric optically active N-substituted-indoline-2-caroboxylic acid which is not esterified;
Step (B): a step in which the composition obtained in the step (A) is mixed with an organic solvent and a basic substance in a presence of water, and allowed to separate into two layers to collect an organic solvent layer containing the optically active N-substituted-indoline-2-carboxylic acid ester and an aqueous layer containing the optically active N-substituted-indoline-2-cabroxylic acid or a salt thereof;
Step (C): a step in which the optically active N-substituted-indoline-2-carboxylic acid ester obtained from the organic solvent layer collected in the step (B) is hydrolyzed to obtain an optically active N-substituted-indoline-2-carboxylic acid; and
Step (D): a step in which the N-substituent of the optically active N-substituted-indoline-2-carboxylic acid obtained in the step (C) is deprotected to obtain an optically active indoline-2-cabroxylic acid.
Step (E): a step in which an N-substituent of the optically active N-substituted-indoline-2-cabroxylic acid or a salt thereof obtained from the aqueous layer collected in the step (B) is deprotected to obtain an optically active indoline-2-cabroxylic acid; 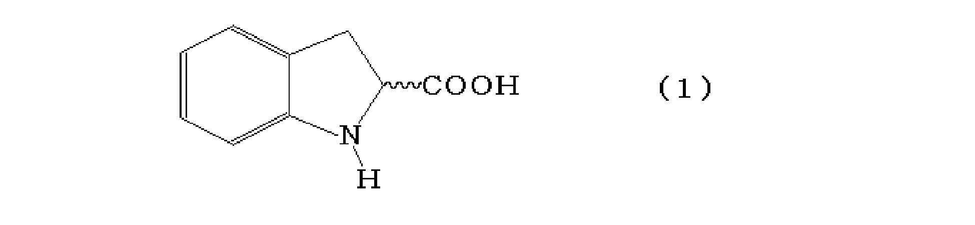 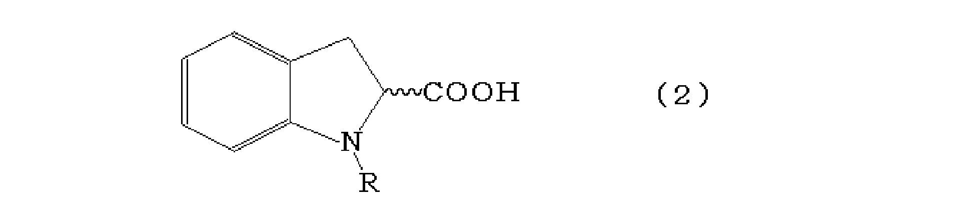
wherein the substituent R represents a formyl group, acetyl group, methoxycarbonyl group, tert-butoxycarbonyl group or benzyloxycarbonyl group.

2. The process for producing an optically active indoline-2-carboxylic acid or a derivative thereof, according to claim 1, wherein the biocatalyst having a stereoselectivity is an esterase.

3. The process for producing an optically active indoline-2-carboxylic acid or a derivative thereof, according to claim 2, wherein the esterase is a lipase.

4. The process for producing an optically active indoline-2-carboxylic acid or a derivative thereof, according to claim 3, wherein the lipase is derived from an yeast which belongs to the genus Candida.

5. The process for producing an optically active indoline-2-carboxylic acid or a derivative thereof, according to claim 1, wherein the alcohol is a primary or secondary alcohol.

6. The process for producing an optically active indoline-2-carboxylic acid or a derivative thereof, according to claim 5, wherein the primary or secondary alcohol is one or more selected form the group consisting of methanol, ethanol, 1-propanol, 2-propanol, 1-butanol, 2-butanol, 2-methyl-1-propanol, 1-pentanol, 2-pentanol and 3-pentanol.

7. The process for producing an optically active indoline-2-carboxylic acid or a derivative thereof, according to claim 5, wherein the alcohol is a methanol.

8. The process for producing an optically active indoline-2-carboxylic acid or a derivative thereof, according to claim 1, wherein the organic solvent is one or more selected from the group consisting of diisopropyl ether, n-butyl ether and ethyl acetate.

9. The process for producing an optically active indoline-2-carboxylic acid or a derivative thereof, according to claim 1, which further comprises a step of subjecting the optically active indoline-2-cabroxylic acid obtained in the step (D) or (E) to an esterification or N-substitution reaction.

10. The process for producing an optically active indoline-2-carboxylic acid or a derivative thereof, according to any one of claims 1 to 9, wherein the optically active indoline-2-carboxylic acid derivative is an optically active indoline-2-carboxylic acid ester or an optically active N-substituted-indoline-2-cabroxylic acid ester.
